# EUROPEAN PATENT APPLICATION

(11) **EP 4 707 409 A1**
(43) Date of publication of application: **11.03.2026**
(21) Application number: 24797423.1
(22) Date of filing: 25.04.2024
(51) Int. Cl.: C12Q 1/6886, G01N 33/574

(54) **NOVEL BLOOD BIOMARKER GULP1 FOR DIAGNOSING LIVER CANCER AND USE THEREOF**

(30) Priority: 27.04.2023 KR 20230055667; 22.04.2024 KR 20240053322
(71) Applicant: Ajou University Industry-Academic Cooperation Foundation, Suwon-si, Gyeonggi-do 16499 (KR)
(72) Inventor: EUN, Jung Woo, Suwon-si Gyeonggi-do 16223 (KR); CHEONG, Jae Youn, Suwon-si Gyeonggi-do 16504 (KR); KIM, Soon Sun, Suwon-si Gyeonggi-do 16517 (KR); CHO, Hyo Jung, Suwon-si Gyeonggi-do 16493 (KR); YOON, Jung Hwan, Seoul 06581 (KR)
(74) Representative: Mewburn Ellis LLP
(86) International application number: PCT/KR2024/005592
(87) International publication number: WO 2024/225769

(57) **Abstract**

The present invention relates to a novel blood biomarker GULP1 for diagnosing liver cancer and use thereof, and to use of GULP1, the expression of which specifically changes in hepatocellular carcinoma, as a biomarker for diagnosing liver cancer. In particular, since GULP1 expression specifically changes in liver cancer, GULP1 can be used as a hepatocellular carcinoma-specific marker, and it was confirmed that, when used alone or in combination with AFP, GULP1 had the effect of diagnosing and predicting hepatocellular carcinoma more specifically and accurately. Therefore, GULP1 discovered in the present invention is highly likely to be used as a target for treating liver cancer.

## Description

### Technical Field

The present disclosure relates to a novel blood biomarker GULP1 for diagnosing a liver cancer and a use thereof.

### Background Art

Liver cancer is a malignant tumor with a poor prognosis, ranked fifth in incidence and second in mortality according to the 2013 Central Cancer Registry statistics. Lacking reliable serum biomarkers for liver cancer, there is an urgent need to discover next-generation serum liver cancer biomarkers for early diagnosis, prognosis, and prediction of treatment response.

Most patients with the liver cancer have underlying cirrhosis, which bears high risks of bleeding during biopsy and poses significant threats. Therefore, the need for liquid biopsy to diagnose liver cancer and obtain genetic information based on blood is gradually increasing. Compared to tissue biopsy that carries significant risks, liquid biopsy is noninvasive and simpler as diagnosis is carried out with blood. Unlike tissue biopsies, blood may be collected multiple times during the treatment process, making it suitable for predicting treatment response or residual cancer during the treatment process.

Since alpha-fetoprotein (AFP), which has been used as a liver cancer diagnostic marker, has a sensitivity of only about 60% for liver cancer diagnosis, with no reliable serum biomarker for liver cancer to date, there is an urgent need to discover next-generation serum liver cancer biomarkers for early diagnosis, prognosis, and prediction of treatment response.

### Disclosure of the Invention

### Technical Goals

An object of the present disclosure is to provide a biomarker composition for diagnosing a liver cancer or predicting liver cancer recurrence or liver cancer metastasis, including GULP1 as an active ingredient.

In addition, another object of the present disclosure is to provide a composition for diagnosing a liver cancer or predicting liver cancer recurrence or liver cancer metastasis, including an agent capable of measuring an expression level of GULP1 in blood as an active ingredient.

In addition, another object of the present disclosure is to provide a kit for diagnosing a liver cancer or predicting liver cancer recurrence or liver cancer metastasis, including the composition.

Moreover, another object of the present disclosure is to provide a method of providing information necessary for diagnosing a liver cancer or predicting liver cancer recurrence or liver cancer metastasis, including measuring an expression level of GULP1 in blood.

Furthermore, another object of the present disclosure is to provide a method of providing information necessary for diagnosing a liver cancer or predicting liver cancer recurrence or liver cancer metastasis, including measuring an expression level of GULP1 and an amount of AFP in blood.

### Technical Solutions

To achieve the above objects, the present disclosure provides a biomarker composition for diagnosing a liver cancer, including GULP1 as an active ingredient.

In addition, the present disclosure provides a biomarker composition for predicting liver cancer recurrence or liver cancer metastasis, including GULP1 as an active ingredient.

In addition, the present disclosure provides a composition for diagnosing a liver cancer, including an agent capable of measuring an expression level of GULP1 in blood as an active ingredient.

In addition, the present disclosure provides a composition for predicting liver cancer recurrence or liver cancer metastasis, including an agent capable of measuring an expression level of GULP1 in blood as an active ingredient.

In addition, the present disclosure provides a kit for diagnosing a liver cancer, including the composition.

In addition, the present disclosure provides a kit for predicting liver cancer recurrence or liver cancer metastasis, including the composition.

In addition, the present disclosure provides a method of providing information necessary for diagnosing a liver cancer, including (1) measuring an expression level of GULP1 from a blood sample isolated from a patient; (2) comparing the measured expression level of GULP1 with that of a control sample; and (3) determining as a liver cancer if the measured expression level of GULP1 is higher than that of the control sample.

In addition, the present disclosure provides a method of providing information necessary for diagnosing a liver cancer, including (1) measuring an expression level of GULP1 and an amount of alpha-fetoprotein (AFP) from a blood sample isolated from a patient; (2) comparing the measured expression level of GULP1 and the measured amount of AFP with those of a control sample; and (3) determining as a liver cancer if the measured expression level of GULP1 and the measured amount of AFP are higher than those of the control sample.

In addition, the present disclosure provides a method of providing information necessary for predicting liver cancer recurrence or liver cancer metastasis, including: (1) measuring an expression level of GULP1 from a blood sample isolated from a liver cancer patient; (2) comparing the measured expression level of GULP1 with that of a control sample; and (3) predicting that there is a high possibility of liver cancer recurrence or liver cancer metastasis when the measured expression level of GULP1 is higher than that of the control sample.

In addition, the present disclosure provides a method of providing information necessary for predicting liver cancer recurrence or liver cancer metastasis, including (1) measuring an expression level of GULP1 and an amount of AFP from a blood sample isolated from a liver cancer patient; (2) comparing the measured expression level of GULP1 and the measured amount of AFP with those of a control sample; and (3) predicting that there is a high possibility of liver cancer recurrence or liver cancer metastasis when the measured expression level of GULP1 and the measured amount of AFP are higher than those of the control sample.

### Advantageous Effects

The present disclosure relates to a novel blood biomarker GULP1 for diagnosing a liver cancer and a use thereof, and to utilization of GULP1, whose expression changes specifically in hepatocellular carcinoma, as a biomarker for diagnosing a liver cancer. In particular, since GULP1 expression changes specifically in liver cancer, it may be used as a marker specific for hepatocellular carcinoma, and it was revealed that it has an effect of diagnosing and predicting hepatocellular carcinoma more specifically and accurately when used alone or in combination with AFP. Accordingly, GULP1 discovered in the present disclosure has a very high possibility of being used as a target for liver cancer treatment.

### Brief Description of Drawings

FIG. 1 shows expression results of GULP1 in three genome datasets.
FIG. 2 shows a result of analyzing expression of GULP1 in 33 carcinomas.
FIG. 3 shows results of a survival rate depending on expression of GULP1.
FIG. 4 shows results of identifying GULP1 expression in HCC through GepLiver DB analysis.
FIG. 5 shows results of identifying GULP1 expression in HCC through spatial transcriptome analysis.
FIG. 6 shows results of scRNA-seq analysis using GepLiver DB.
FIG. 7 shows results of scRNA-seq analysis using scAtlasLC.
FIG. 8 shows a result of an expression ratio of GULP1 in tissues of liver cancer patients who underwent hepatectomy.
FIG. 9 shows results of an expression ratio of GULP1 and a ratio in patients with recurrence depending on recurrence/metastasis.
FIG. 10 shows results of comparing GULP1 protein concentrations in the blood of normal and liver cancer patients.
FIG. 11 shows a protein concentration of GULP1 and AUROC results in a liver disease at each stage.
FIG. 12 shows results of comparative ROC analysis with AFP in patients at each disease stage of mUICC.
FIG. 13 shows results of ROC analysis according to a combination of AFP and GULP1.
FIG. 14 shows a result of positive rate for AFP and GULP1 at each disease stage.
FIG. 15 shows a result of a liver cancer diagnosis ratio of AFP and GULP1.
FIG. 16 shows results of a degree of protein concentration of GULP1 according to the recurrence and metastasis of a liver cancer.
FIG. 17 shows results of analyzing a disease-free survival rate.

### Best Mode for Carrying Out the Invention

The present disclosure provides a biomarker composition for diagnosing a liver cancer, including GULP1 as an active ingredient.

In addition, the present disclosure provides a biomarker composition for predicting liver cancer recurrence or liver cancer metastasis, including GULP1 as an active ingredient.

The term "diagnosis" as used herein includes determining the susceptibility of a subject to a particular disease or condition, determining whether a subject currently has a particular disease or condition, determining the prognosis of a subject with a particular disease or condition, or therametrics (e.g., monitoring the condition of a subject to provide information about the efficacy of a treatment).

The term "prediction" as used herein refers to a process of predicting an outcome of treatment for a pathological condition by collecting data on the progress of the pathological condition and the treatment process. For the purposes of the present disclosure, the prediction may be interpreted as determining the possibility of recurrence or metastasis after liver cancer treatment or the possibility of death thereby, but is not limited thereto.

In addition, the present disclosure provides a composition for diagnosing a liver cancer, including an agent capable of measuring an expression level of GULP1 in blood as an active ingredient.

Preferably, the liver cancer may be an early liver cancer, but is not limited thereto.

Preferably, the agent capable of measuring an expression level may be, but is not limited to, a primer or probe that specifically binds to a gene of the GULP1, and an antibody, peptide, aptamer, or compound that specifically binds to a protein of the GULP1.

In addition, the present disclosure provides a kit for diagnosing a liver cancer, including the composition.

In addition, the present disclosure provides a composition for predicting liver cancer recurrence or liver cancer metastasis, including an agent capable of measuring an expression level of GULP1 in blood as an active ingredient.

Preferably, the agent capable of measuring an expression level may be, but is not limited to, a primer or probe that specifically binds to a gene of the GULP1, and an antibody, peptide, aptamer, or compound that specifically binds to a protein of the GULP1.

In addition, the present disclosure provides a kit for predicting liver cancer recurrence or liver cancer metastasis, including the composition.

The term "primer" as used herein refers to a short nucleic acid sequence having a short free 3' hydroxyl group, capable of forming base pairs with a complementary template and serving as an initiation site for duplication of the template strand. The primer may initiate DNA synthesis in the presence of a polymerization reagent (i.e., DNA polymerase or reverse transcriptase) and four different nucleoside triphosphates in an appropriate buffer and temperature. PCR conditions and lengths of sense and antisense primers may be appropriately selected according to techniques known in the art.

As used herein, the term "probe" refers to a nucleic acid fragment in a length ranging from a few bases to several hundred bases, such as RNA or DNA, with a capability of specifically binding to mRNA, and it enables detection of the presence of a specific mRNA and an expression level as it is labeled. Probes may be produced in the form of oligonucleotide probes, single-strand DNA probes, double-strand DNA probes, and RNA probes. Selection of appropriate probes and hybridization conditions may be appropriately selected according to techniques known in the art.

The term "antibody" as used herein is a term known in the art and refers to a specific immunoglobulin directed against an antigenic site. The antibody as used herein refers to an antibody that specifically binds to the biomarker of the present disclosure, and the antibody may be manufactured according to a conventional method in the relevant technical field. The form of the antibody includes polyclonal antibodies or monoclonal antibodies, and all immunoglobulin antibodies are included. The antibody refers to a complete form having two full-length light chains and two full-length heavy chains. Additionally, the antibodies include special antibodies such as humanized antibodies.

In addition, the kit of the present disclosure may include an antibody that specifically binds to a marker component, a secondary antibody conjugate to which a label that develops color by reaction with a substrate is conjugated, a chromogenic substrate solution that reacts with the label, a washing solution, and an enzyme reaction stop solution, and may be manufactured into a plurality of separate packaging or compartments containing the reagent components used.

The term "peptide" as used herein has a high binding affinity to a target substance without undergoing denaturation even when treated with heat or chemicals. Additionally, owing to its small molecular size, it may be attached to other proteins to be used as a fusion protein. Specifically, since it is used by being attached to a polymer protein chain, it may be used as a diagnostic kit and drug delivery material.

The term "aptamer" as used herein refers to a type of polynucleotide composed of a special type of single-stranded nucleic acid (DNA, RNA, or modified nucleic acid) that has a stable tertiary structure in itself with characteristics of being able to bind to a target molecule with high affinity and specificity. As described above, aptamers may be used as a substitute for antibodies since they are able to specifically bind to antigenic substances in the same way as antibodies, ensures higher stability than proteins and simple structures, and are composed of easily synthesizable polynucleotides.

In addition, the present disclosure provides a method of providing information necessary for diagnosing a liver cancer, including (1) measuring an expression level of GULP1 from a blood sample isolated from a patient; (2) comparing the measured expression level of GULP1 with a control sample; and (3) determining as a liver cancer if the measured expression level of GULP1 is higher than that of the control sample.

In addition, the present disclosure provides a method of providing information necessary for diagnosing a liver cancer, including (1) measuring an expression level of GULP1 and an amount of alpha-fetoprotein (AFP) from a blood sample isolated from a patient; (2) comparing the measured expression level of GULP1 and the measured amount of AFP with those of a control sample; and (3) determining as a liver cancer if the measured expression level of GULP1 and the amount of AFP are higher than those of the control sample.

Preferably, the liver cancer may be an early liver cancer, but is not limited thereto.

In addition, the present disclosure provides a method of providing information necessary for predicting liver cancer recurrence or liver cancer metastasis, including: (1) measuring an expression level of GULP1 from a blood sample isolated from a liver cancer patient; (2) comparing the measured expression level of GULP1 with a control sample; and (3) predicting that there is a high possibility of liver cancer recurrence or liver cancer metastasis when the measured expression level of GULP1 is higher than that of the control sample.

In addition, the present disclosure provides a method of providing information necessary for predicting liver cancer recurrence or liver cancer metastasis, including (1) measuring an expression level of GULP1 and an amount of AFP from a blood sample isolated from a liver cancer patient; (2) comparing the measured expression level of GULP1 and the measured amount of AFP with those of a control sample; and (3) predicting that there is a high possibility of liver cancer recurrence or liver cancer metastasis when the measured expression level of GULP1 and the measured amount of AFP are higher than those of the control sample.

In the present disclosure, the method of measuring the expression level of GULP1 may use, but is not limited to, RT-PCR, competitive RT-PCR, real-time RT-PCR, RNase protection assay (RPA), Northern blotting, DNA chip, Western blot, enzyme linked immunosorbent assay (ELISA), radioimmunoassay (RIA), radioimmunodiffusion, Ouchterlony immunodiffusion, rocket immunoelectrophoresis, tissue immunostaining, immunoprecipitation assay, complement fixation assay, FACS, or protein chip.

### Modes for Carrying Out the Invention

Hereinafter, the present disclosure will be described in detail according to examples that do not limit the present disclosure. It should be noted that the following examples of the present disclosure are only intended to illustrate the present disclosure and do not limit or restrict the scope of the present disclosure. Therefore, what those skilled in the art to which the present disclosure pertains may easily infer from the detailed description and examples of the present disclosure is interpreted as falling within the scope of the present disclosure.

### <Experimental Example>

The following Experimental Examples are intended to provide Experimental Examples commonly applied to each Example according to the present disclosure.

### 1. Identification of candidate markers associated with liver cancer recurrence from genomic data of liver cancer patients who underwent hepatectomy.

A total of three genomic data sets (GSE14520, GSE114564, TCGA_LIHC) containing information on liver cancer patients who underwent hepatectomy were selected followed by hepatectomy, and then patients were classified into patients with recurrence (recurrent group) and those without recurrence for more than 2 years (non-recurrent group) to be used for analysis.

### 2. Collection of tissues and clinical information from liver cancer patients who underwent hepatectomy

Liver cancer tissues and surrounding normal tissues were donated from 86 liver cancer patients who underwent hepatectomy at the Human Genome Resource Bank in Ajou University Hospital.

### 3. Collection of blood and clinical information from normal/liver disease cohort

After passing the IRB review at Ajou University Hospital, blood from 30 normal individuals and 51 liver cancer patients was secured as a test cohort through the Human Resources Bank. The validation cohort included 34 patients with chronic hepatitis and 33 patients with cirrhosis, and 47 patients with recurrence of liver cancer at the time of blood sampling and 47 non-recurrence patients were secured. Finally, blood samples were obtained from 7 recurrent and 7 non-recurrent liver cancer patients at Seoul National University Hospital as a cohort from another hospital. Therefore, total blood samples used in the study were 97 non-hepatic cancer samples and 159 hepatic cancer samples, and GULP1 was evaluated in 256 blood samples.

Clinical information was obtained from the Human Resource Center at Ajou University Hospital, including recurrence of liver cancer, metastasis, surgery date, time of recurrence, last estimated date, and survival.

### 4. RNA extraction from tissues

RNA from tissues was extracted using QIAzol Lysis Reagent (Cat#79306).

### 5. Measurement of GULP1 expression level in tissues via qRT-PCR analysis

cDNA synthesis was performed using the PrimeScript^{™} RT Master Mix (Perfect Real Time) (Cat #RR036A) (TaKaRa) kit.

Tissue RNA and RNase Free Water were combined to make 8ul, and 2 ul of PrimeScript^{™} RT Master Mix was added to make a total of 10 ul for cDNA synthesis.

cDNA conditions were set as follows.
A. Stage 1: 37°C, 15 minutes
B. Stage 2: 85°C, 4°C after 5 seconds, -ing

qRT-PCR was performed using primers with the sequences below. The primers used here were purchased from M.biotech (Hanam, Korea). qRT-PCR was performed using 5 ul of qPCR Master Mix (2X, High ROX) (Gendepot, Cat #Q5602) in a total volume of 10 ul.

**TABLE 1**

| Gene | Accession No. | Forward sequence | Reverse sequence |
|---|---|---|---|
| GULP1 | NM_016315.4 | 5'- AACGGGACCTGTTTG G AGCA-3' | 5'- ACCCCTCCTGCATCTC ATCT-3' |
| HMBS | NM_001024382.2 | 5'-GGAGGGCAGAAGGAAG AAAACAG-3' | 5'-CACTGTCCGTCTGTAT GCGAG-3' |

qRT-PCR conditions were set as follows.
A. Stage 1: 95°C, 2 minutes
B. Stage 2: 95°C, 15 seconds
C. : 58°C, 34 seconds
D. : 72°C, 30 seconds
E. Repeat Stage 2 for 40 cycles

### 6. Evaluation of the blood cohort via ELISA

To determine whether serum GULP1 has the potential to act as a biomarker, the concentration of serum GULP1 was measured using the GULP1 ELISA kit (assaygenie) as follows.

The collected patient's whole blood was centrifuged at 3,000 rpm for 30 minutes to separate the serum, which was then stored at -80°C immediately after separation. The frozen serum was thawed on ice and then diluted 1/10 with a sample diluent before use.

A 96-well microtiter plate, which was blocked after coating with a monoclonal antibody against a protein of the GULP1 using a sandwich enzyme-linked immunosorbent assay, was washed twice with PBS solution containing 0.05% Tween 20 (PBS-T). The recombinant GULP1 protein was diluted to 2000 pg/ml, 100 µl was added by serial dilution, the serum sample diluted to 1/10 with the sample diluent was added to each well of a 96-well microtiter plate followed by a reaction at 37°C for 1.5 hours, and washing was performed three times using PBS-T solution.

100 µl of biotin detection antibody was added to each well and then reacted at 37°C for 1 hour, followed by washing three times using PBS-T solution.

After washing, 100 µl of HRP-Streptavidin Conjugate solution was added to each well and reacted at 37°C for 30 minutes, followed by washing 5 times with PBS-T, and finally, 90 µl of TMB solution was added to each well for color development at 37°C for 20 minutes.

After the color development was completed, 50 µl of color stop solution was added to stop the reaction, and the absorbance of the sample was measured using a measuring device (ELISA reader, Promega) at a wavelength of 450 nm.

### 7. Statistical analysis of results

Statistical analysis was performed using MedCalc statistical software, GraphPad, and SPSS v22 analysis programs.

### <Example 1> Identification of GULP1, a specific candidate marker associated with liver cancer recurrence

As a result of analysis, GULP1 having higher expression in the recurrent group compared to the non-recurrent group was identified in all three datasets.

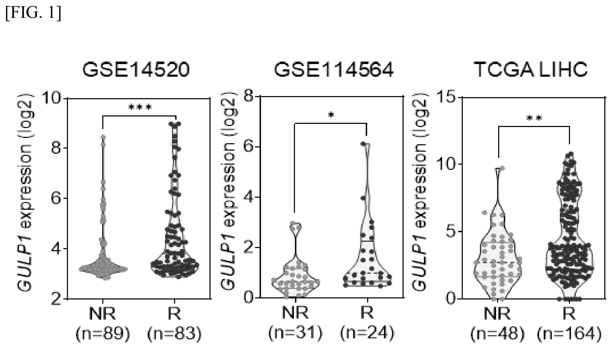

GULP1 was found to have statistically significantly higher expression in the recurrent group compared to the non-recurrent group in all three datasets (FIG. 1).

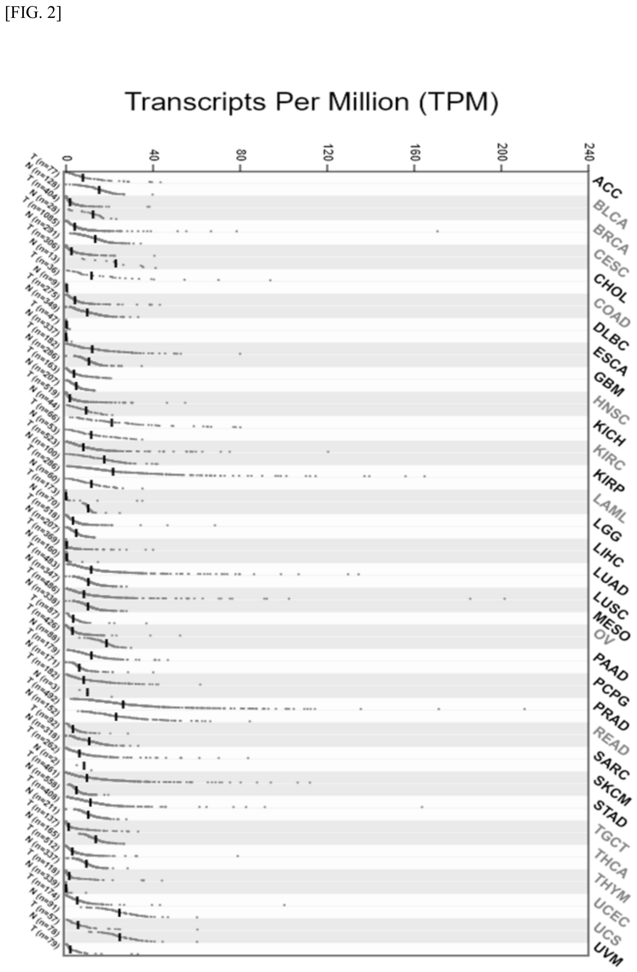

When the expression of GULP1 was analyzed for a total of 33 carcinomas using TCGA Pan-cancer data, GULP1 was rather suppressed in tumors in most carcinomas (green), such that it is predicted to be a tumor suppressor (FIG. 2).

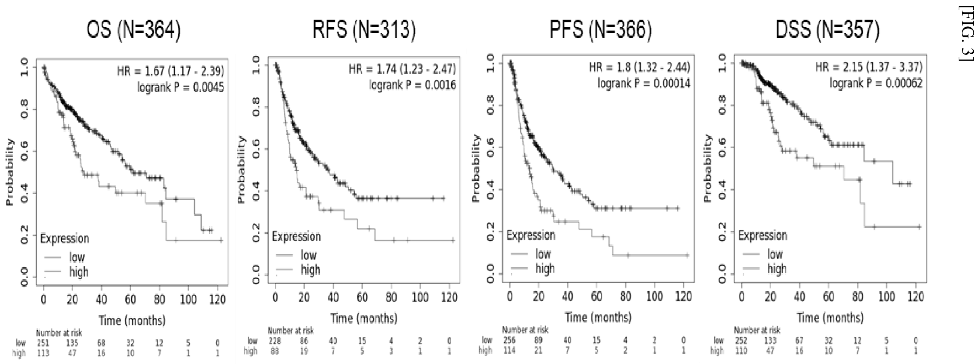

However, in liver cancer, it was analyzed that the prognosis was worse in OS, RFS, PFS, and DSS in the patient group with high expression of GULP1, showing that GULP1 was specifically overexpressed in the liver cancer (FIG. 3).

### <Example 2> Evaluation of specificity of GULP1 in tissues

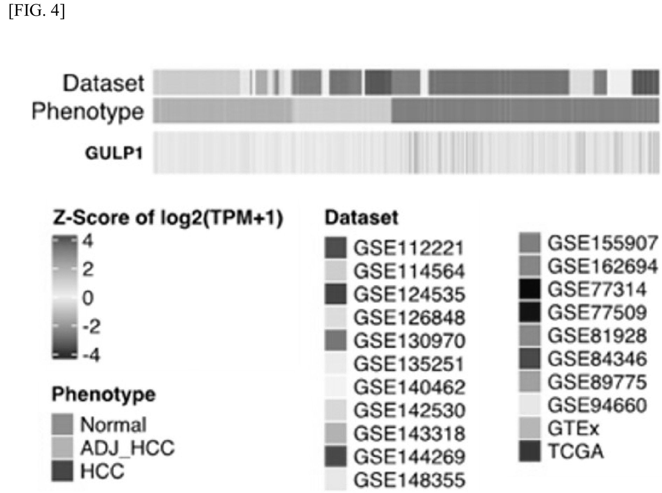

The expression of GULP1 in different stages of HCC was further investigated using the GepLiver DB dataset and spatial transcriptome analysis. First, as a result of analyzing the heatmap generated from the GepLiver DB dataset, there were stark differences between GULP1 expressions in normal, adjacent (HCC-adjacent tissue, ADJ_HCC), and HCC liver tissues, with a marked increase in GULP1 levels in HCC samples. This expression pattern was consistent across all 22 datasets, presenting the potential of GULP1 as a biomarker for HCC (FIG. 4).

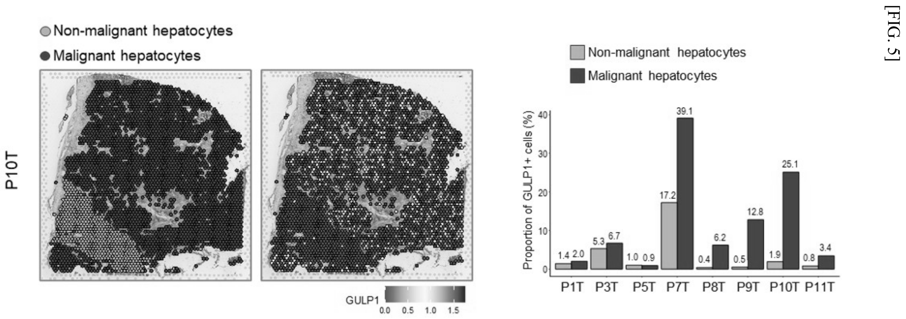

Additionally, spatial transcriptome analysis showed that GULP1 expression was remarkably increased within malignant hepatocytes, indirectly presenting its role within the tumor microenvironment. Although the proportion of GULP1-positive cells varied in the analyzed tumor tissues from different patients, a consistent increase in GULP1 expression was observed, suggesting an association with HCC (FIG. 5).

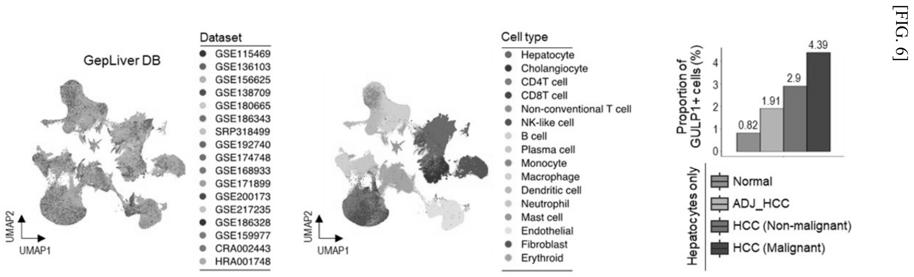

Moreover, analysis of scRNA-seq data in GepLiver DB also showed diverse expression patterns of GULP1. When hepatocyte-specific GULP1 expression was observed in HCC tissues, a significant portion of hepatocytes were found to be GULP1 positive. A gradual increase in GULP1 expression was observed during the progression from normal liver to malignant state (FIG. 6).

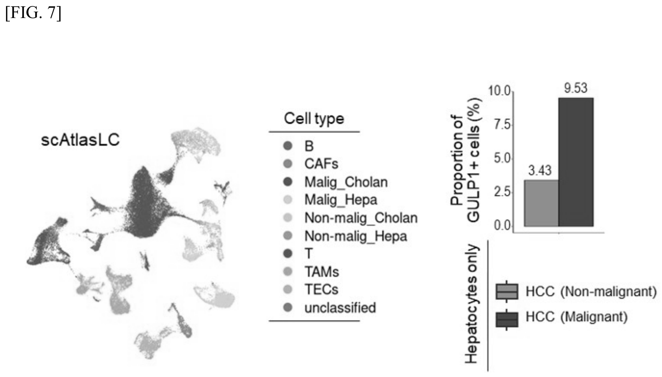

Similarly, UMAP quantification analysis in the scAtlasLC database demonstrates increased expression of genes in HCC, highlighting the importance in the context of liver cancer pathology. These results demonstrate that GULP1 expression is increased in HCC, presenting its potential role as a key biomarker in predicting the diagnosis of HCC (FIG. 7).

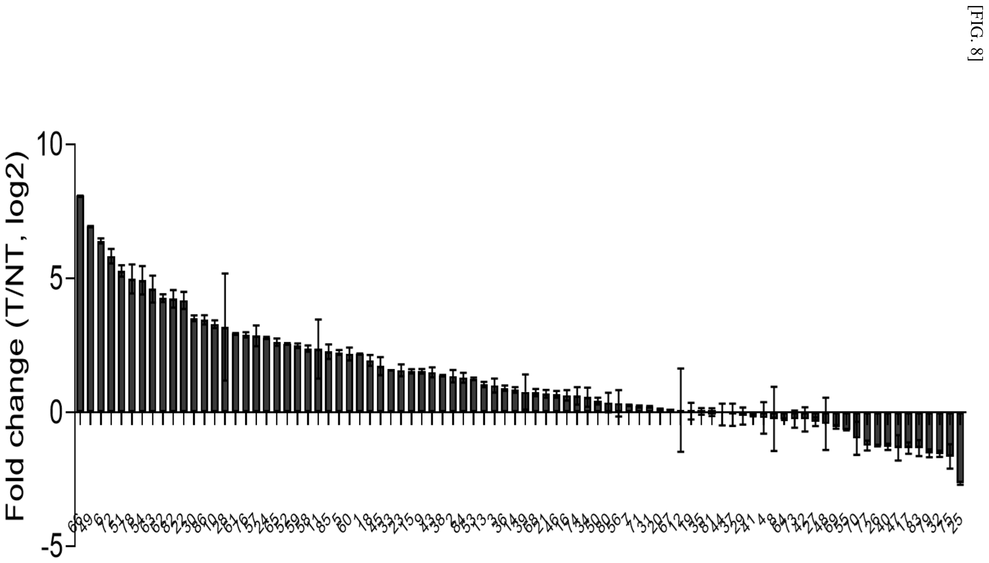

As a result of observing the expression of GULP1 in 86 pairs of normal surrounding tissues and liver cancer tissues donated from the Human Genome Resource Bank at Ajou University Hospital, it was determined that GULP1 expression was higher in liver cancer tissues than in normal surrounding tissues in approximately 62.8% (>1.2 fold) of patients (FIG. 8).

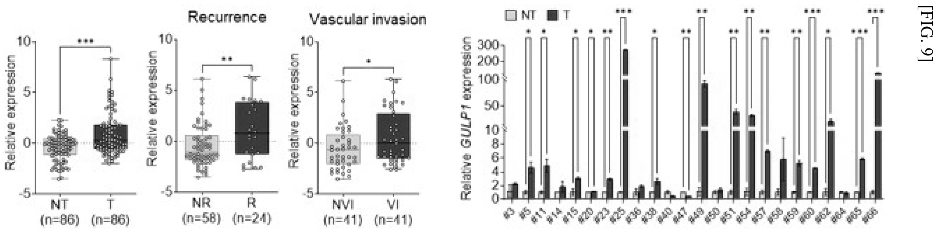

Among 86 pairs of patients, as a result of comparing expression according to the presence of recurrence and microvascular invasion, it was found that expression in patients with vascular invasion (VI) was high among patients with recurrence (R, recur). In addition, as a result of selecting 24 patients with recurrence to detect GULP1 expression in normal surrounding tissues and liver cancer tissues, it was found that GULP1 expression was significantly high in approximately 70.8% of patients (FIG. 9).

### <Example 3> Evaluation of the specificity of GULP1 as a blood marker for liver cancer patients

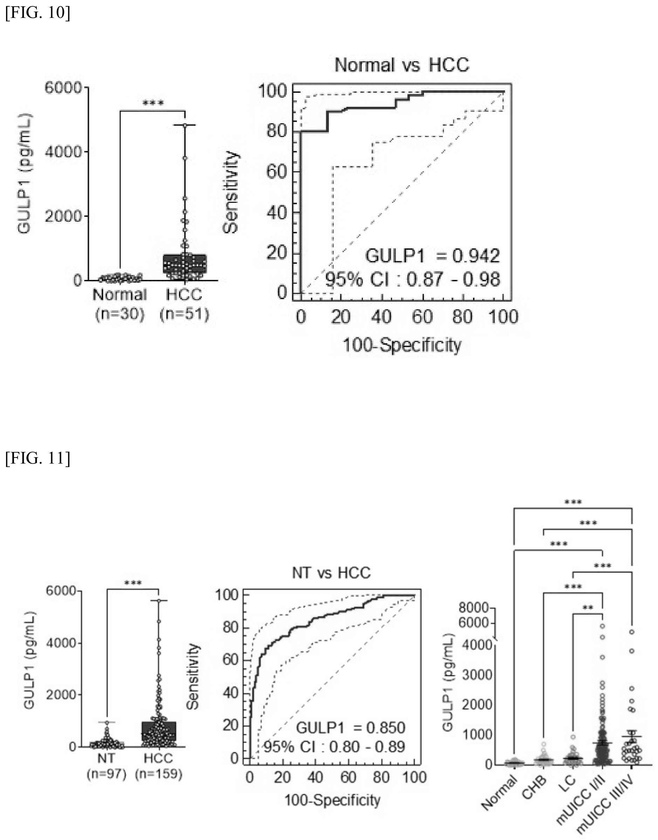

When protein concentrations were measured via ELISA in the serum of 30 normal individuals and 51 liver cancer patients to evaluate GULP1 as a noninvasive diagnostic marker, it was revealed that very low concentration of expression was observed in the normal group, but liver cancer patients showed relatively high expression. In the diagnosis of liver cancer, the ROC analysis results showed that serum GULP1 had a very high diagnostic ability with an AUC of 0.942 (95% CI: 0.87-0.98) for liver cancer diagnosis (FIG. 10).

### <Example 4> Evaluation of the diagnostic ability of GULP1 in a liver disease cohort

In normal individuals, AFP, the existing liver cancer blood marker, was measured at very low levels, so verification of the marker's expression in patients with hepatitis and cirrhosis is also essential to identify liver cancer specificity. Therefore, evaluation was conducted on GULP1 in a total of 97 non-hepatic cancer patients and 159 liver cancer patients, including blood samples from patients with hepatitis and cirrhosis and adding approximately 100 liver cancer patients.

Compared to the non-liver cancer group, the liver cancer group showed a higher concentration of GULP1, and the ROC analysis results for liver cancer diagnosis showed that serum GULP1 had an AUC of 0.850 (95% CI: 0.80-0.89) for liver cancer diagnosis, which was evaluated to be very high even when the liver disease group was included. The cut-off value for liver cancer diagnosis was found to be 340 pg/mL (FIG. 11).

As shown in FIG. 11, in order to specifically evaluate the diagnostic ability of GULP1, the patients were divided into normal liver (n=30), chronic hepatitis (n=34), cirrhosis (n=33), mUICC I/II (n=129), and mUICC III/IV (n=30) to determine the protein concentration of GULP1. It was found that the expression of serum GULP1 in liver cancer patients was statistically significantly increased compared to non-liver cancer patients (NL, CH, LC) (ANOVA-test, *P<0.05, **P<0.01, ***P<0.001).

### <Example 5> Validation results of serum GULP1 as an early liver cancer marker

To determine whether early liver cancer was detected, the mUICC stage was divided to measure the AUC. It was attempted to determine statistically significant superiority through analysis involving comparison with AFP, an existing liver cancer serum marker.

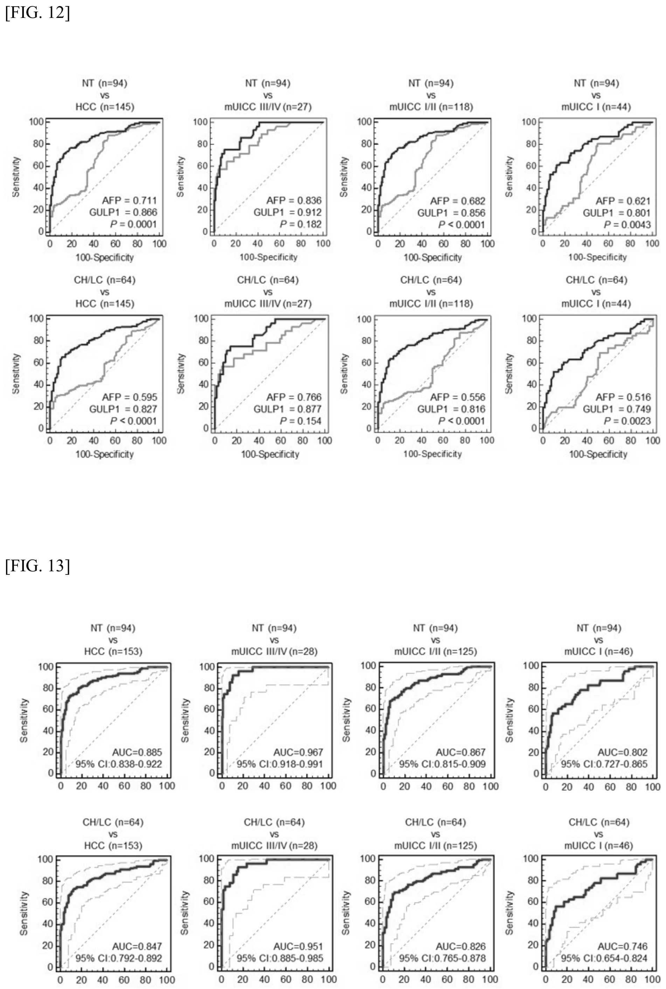

As shown in FIG. 12, in the diagnosis of liver cancer in the entire liver cancer cohort, AFP had an AUC of 0.711, and GULP1 had an AUC of 0.866, and when compared in the high-risk groups for liver cancer, CH and LC, excluding normal, the AUC of AFP was 0.595, and that of GULP1 was 0.827, showing a significant difference (P<0.0001).

In the liver cancer diagnosis in the advanced liver cancer cohort including mUICC III and IV, AFP had an AUC of 0.836 and GULP1 showed an AUC of 0.912, and when compared in the high-risk groups of liver cancer, CH and LC, excluding normal, the AUC of AFP was 0.766 and that of GULP1 was 0.877, indicating that GULP1 was higher, but was not statistically significant.

In the liver cancer diagnosis in the early liver cancer cohort including mUICC I and II, AFP had an AUC of 0.682 and GULP1 had an AUC of 0.856, and when compared in the high-risk groups of liver cancer, CH and LC, excluding normal, an AUC of AFP was 0.556 and that of GULP1 was 0.816, showing a relatively superior diagnostic ability (P<0.0001).

In the diagnosis of liver cancer in the earliest stage mUICC I with a tumor size of 2 cm or less, AFP had an AUC of 0.621, and GULP1 had an AUC of 0.801, and when compared in high-risk groups for liver cancer, CH and LC, excluding normal, the AUC of AFP was 0.516, and that of GULP1 was 0.749, showing that the diagnostic accuracy for AFP dropped sharply, while that of GULP1 was maintained.

### <Example 6> Evaluation of the diagnostic ability for liver cancer according to the combination of serum AFP and GULP1.

The diagnostic ability for liver cancer was verified by calculating the probability index through binary logistic analysis of concentration values of AFP and GULP 1 using SPSS.

As shown in FIG. 13, in the diagnosis of liver cancer in the entire liver cancer cohort, AFP+GULP1 showed an AUC value of 0.885, which had higher AUC values than AFP=0.711 and GULP1=0.866. When compared in CH and LC, which are high-risk groups of liver cancer, AFP+GULP1 had an AUC of 0.847, which was evaluated to be higher than AFP=0.595 and GULP1=0.827.

In the diagnosis of liver cancer in an advanced liver cancer cohort including mUICC III and IV, AFP+GULP1 showed an AUC of 0.967, which had higher values than AFP=0.836 and GULP1=0.912. When compared in CH and LC, which are high-risk groups of liver cancer, AFP+GULP1 had an AUC of 0.951, which was evaluated to be higher than AFP=0.766 and GULP1=0.877.

In the liver cancer diagnosis in the early liver cancer cohort including mUICC I and II, AFP+GULP1 showed an AUC of 0.867, which had higher values than AFP=0.682 and GULP1=0.856. When compared in CH and LC, which are high-risk groups for liver cancer, AFP+GULP1 had an AUC of 0.826, which was evaluated to be higher than AFP=0.556 and GULP1=0.816.

In the diagnosis of liver cancer in the earliest stage mUICC I with a tumor size of 2 cm or less, AFP+GULP1 showed an AUC of 0.802, which had almost similar AUC value to that of GULP1 alone, compared to AFP=0.621 and GULP1=0.801. In CH and LC, the high-risk groups of liver cancer, AFP+GULP1 had an AUC of 0.746, indicating that GULP1 alone showed the best indicator in mUICC I compared to AFP=0.516 and GULP1=0.749.

### <Example 7> Comparison of positive/negative ratios of serum AFP and serum GULP1 in each disease group

In order to compare the positive/negative ratios of serum AFP and serum GULP1 in each disease group, comparative analysis was conducted by applying cutoff=20ng/mL for AFP and cutoff=340pg/mL for GULP1.

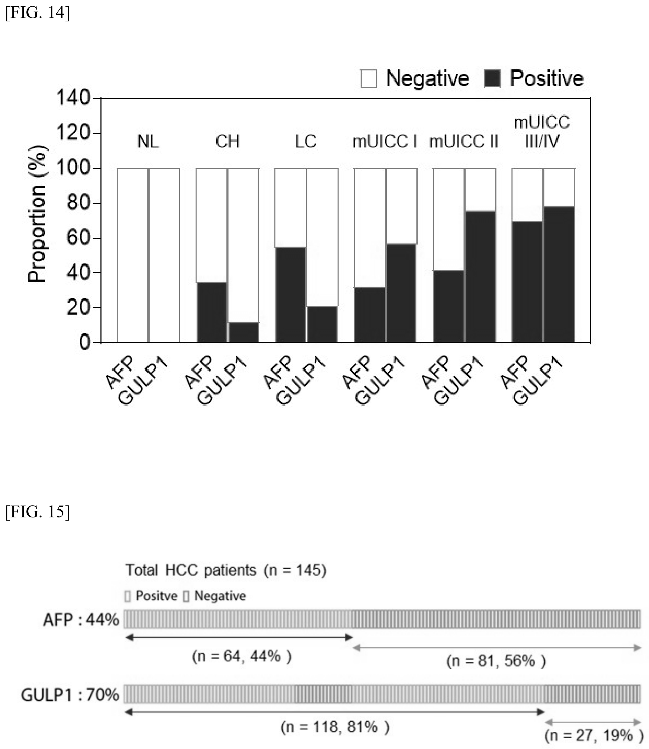

As shown in FIG. 14, in the case of normal liver (NL), both AFP and GULP1 showed a positivity rate of 0%; in the case of chronic hepatitis (CH), AFP showed a positivity rate of 35%, and GULP1 showed a positivity rate of 12%; and in the case of liver cirrhosis (LC), AFP showed a positivity rate of 55%, and GULP1 showed a positivity rate of 21%.

In liver cancer, AFP showed a positivity rate of 32% and GULP1 showed a positivity rate of 57% in mUICC I; AFP showed a positivity rate of 42% and GULP1 showed a positivity rate of 76% in mUICC II; and AFP showed a positivity rate of 70% and GULP1 showed a positivity rate of 78% in mUICC III/IV.

Therefore, in the non-liver cancer group, the rate of GULP1 being positive was found to be much lower than that of AFP, and in the case of liver cancer, it was evaluated that it could detect much higher levels than AFP, especially in the early stage of liver cancer, mUICC I/II.

### <Example 8> Degree of improvement in the diagnostic ability depending on the combination of serum AFP and serum GULP1 in liver cancer patients

When the liver cancer diagnosis rate for AFP and GULP1 was evaluated in the 145 patients with liver cancer, it was evaluated as 44% for AFP and 70% for GULP1. It was found that when AFP and GULP1 were used as combined diagnostic markers, the diagnostic rate increased to 81% (FIG. 15).

Therefore, GULP1 showed potential as a diagnostic marker alone or in combination with AFP.

### <Example 9> Evaluation of the clinical efficacy of serum GULP1 in liver cancer patients

Clinical information on recurrence and metastasis was obtained from all liver cancer patients, and the degree of protein concentration of GULP1 was compared and evaluated.

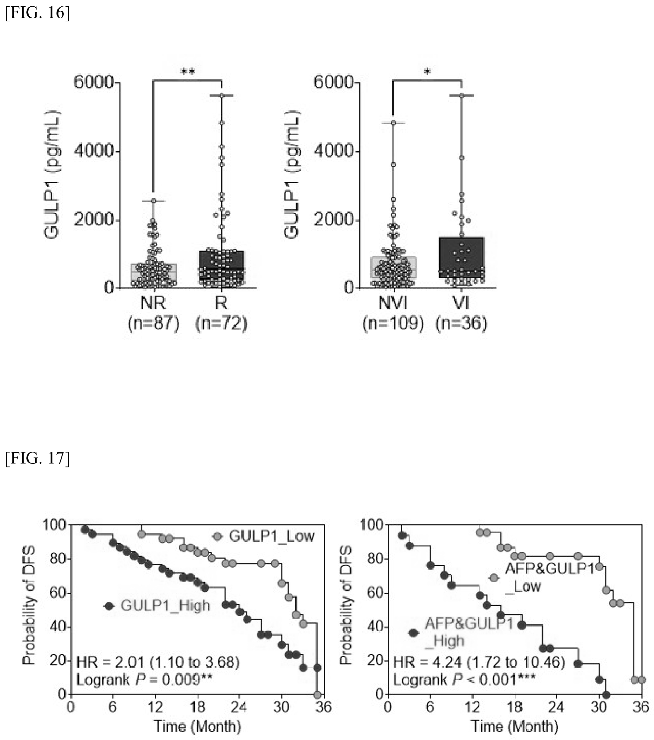

As shown in FIG. 16, when comparing the protein concentration of GULP1 in patients with recurrence (72 patients in total) and those without (87 patients in total) among liver cancer patients, it was determined that the expression level of GULP1 was statistically significantly higher in patients with recurrence (non-recurrence average 616, recurrence average 1000 pg/mL, P=0.006).

Similarly, when comparing the protein concentration of GULP1 in patients with metastasis (36 patients in total) and those without (109 patients in total), it was determined that the expression level of GULP1 was statistically significantly higher in patients with metastasis (non-recurrence average 717.7, recurrence average 1063 pg/mL, P=0.04).

As of the present time, clinical information was tracked according to the presence or absence of recurrence, and disease-free survival (DFS) was analyzed.

As shown in FIG. 17, when the survival rate was expressed with a Kaplan-Meier survival curve for the high patient group (n=39) and the low patient group (n=39) based on the median value (600 pg/mL) of the level of GULP1 protein concentration according to the recurrence of liver cancer patients within 3 years, the hazard ratio (HR) in the high GULP1 patient group was evaluated as 2.01 (95% CI=1.10-3.68, log-rank P=0.009).

When the concentration standards for AFP and GULP1 were set as AFP=20ng/mL and GULP1=600pg/mL to express with the Kaplan-Meier survival curves for the group of patients with high levels of both markers (n=17) and the group of patients with low levels of both markers (n=24), the hazard ratio (HR) in the group of patients with high levels of both AFP and GULP1 was evaluated as 4.24 (95% CI=1.72-10.46, log-rank P < 0.001).

In conclusion, the present disclosure relates to a liver cancer diagnostic ability using GULP1 overexpressed in the blood of liver cancer patients, which is capable of accurately diagnosing early liver cancer by showing considerably excellent sensitivity and specificity even with a small amount of blood, such that it may be considered to be effective in terms of diagnostic accuracy, simplicity, and cost-effectiveness.

In addition, the present disclosure discovered GULP1 that is specifically overexpressed in liver cancer cells and selected GULP1 by first verifying it in public omics data, with an excellent diagnostic accuracy by verifying the clinical efficacy of the selected GULP1 in an independent liver disease cohort, and in a situation where the detection rate of early liver cancer using ultrasound is 60-80%, which is below expectations, while AFP, known as a serum biomarker, also plays a minimal role in early liver cancer, the present disclosure shows surprising liver cancer diagnostic accuracy using a small amount of blood, such that it is expected that the present disclosure will play an innovative role in screening tests for high-risk liver cancer groups in the future.

While a specific part of the present disclosure has been described in detail above, it is clear for those skilled in the art that this specific description is merely preferred example embodiments, and the scope of the present disclosure is not limited thereby. In other words, the substantial scope of the present disclosure is defined by the appended claims and their equivalents.

## Claims

1. A biomarker composition for diagnosing a liver cancer, comprising GULP1 as an active ingredient.

2. A biomarker composition for predicting liver cancer recurrence or liver cancer metastasis, comprising GULP1 as an active ingredient.

3. A composition for diagnosing a liver cancer, comprising an agent capable of measuring an expression level of GULP1 in blood as an active ingredient.

4. The composition of claim 3, wherein the liver cancer is an early liver cancer.

5. The composition of claim 3, wherein the agent capable of measuring the expression level is a primer or probe that specifically binds to a gene of the GULP1, or an antibody, peptide, aptamer, or compound that specifically binds to a protein of the GULP1.

6. A kit for diagnosing a liver cancer, comprising a composition according to any one of claims 3 to 5.

7. A composition for predicting liver cancer recurrence or liver cancer metastasis, comprising an agent capable of measuring an expression level of GULP1 in blood as an active ingredient.

8. The composition of claim 7, wherein the agent capable of measuring the expression level is a primer or probe that specifically binds to a gene of the GULP1, or an antibody, peptide, aptamer, or compound that specifically binds to a protein of the GULP1.

9. A kit for predicting liver cancer recurrence or liver cancer metastasis, comprising the composition according to claim 7 or 8.

10. A method of providing information necessary for diagnosing a liver cancer, the method comprising:
(1) measuring an expression level of GULP1 from a blood sample isolated from a patient;
(2) comparing the measured expression level of GULP1 with that of a control sample; and
(3) determining as a liver cancer if the measured expression level of GULP1 is higher than that of the control sample.

11. A method of providing information necessary for diagnosing a liver cancer, the method comprising:
(1) measuring an expression level of GULP1 and an amount of alpha-fetoprotein (AFP) from a blood sample isolated from a patient;
(2) comparing the measured expression level of GULP1 and the measured amount of AFP with those of a control sample; and
(3) determining as a liver cancer if the measured expression level of GULP1 and the measured amount of AFP are higher than those of the control sample.

12. The method of claim 10 or 11, wherein the liver cancer is an early liver cancer.

13. A method of providing information necessary for predicting liver cancer recurrence or liver cancer metastasis, the method comprising:
(1) measuring an expression level of GULP1 from a blood sample isolated from a liver cancer patient;
(2) comparing the measured expression level of GULP1 with that of a control sample; and
(3) predicting that there is a high possibility of liver cancer recurrence or liver cancer metastasis when the measured expression level of GULP1 is higher than that of the control sample.

14. A method of providing information necessary for predicting liver cancer recurrence or liver cancer metastasis, the method comprising:
(1) measuring an expression level of GULP1 and an amount of AFP from a blood sample isolated from a liver cancer patient;
(2) comparing the measured expression level of GULP1 and the measured amount of AFP with those of a control sample; and
(3) predicting that there is a high possibility of liver cancer recurrence or liver cancer metastasis when the measured expression level of GULP1 and the measured amount of AFP are higher than those of the control sample.
